# EUROPÄISCHE PATENTSCHRIFT

(11) **EP 0 452 349 B1**
(45) Veröffentlichungstag und Bekanntmachung des Hinweises auf die Patenterteilung: **01.06.1994**
(21) Anmeldenummer: 90900791.6
(22) Anmeldetag: 06.12.1989
(51) Int. Cl.: C07C 231/10, C07C 233/36, A61K 7/00, C11D 1/10

(54) **VERFAHREN ZUR HERSTELLUNG VON AMPHOTEREN GRENZFLÄCHENAKTIVEN IMIDAZOLINDERIVATEN**
PROCESS FOR PRODUCING AMPHOTERIC SURFACE-ACTIVE IMIDAZOLINE DERIVATIVES
PROCEDE DE PRODUCTION DE DERIVES D'IMIDAZOLINE AMPHOTERES TENSIO-ACTIFS

(30) Priorität: 15.12.1988 ES 8803808
(43) Veröffentlichungstag der Anmeldung: 23.10.1991
(73) Patentinhaber: Henkel Kommanditgesellschaft auf Aktien, 40191 Düsseldorf (DE)
(72) Erfinder: TRIUS, D. Antonio, E-Valldoreix Sant Cugat (ES); HUMBERT, Miquel, E-08902 Sabadell (ES); CUADRADO, Francisco, E-08013 Barcelona (ES); BIGORRA, Joaquin, E-08203 Sabadell (ES); POMARES, Jesus, E-08028 Barcelona (ES); PLOOG, Uwe, D-5657 Haan (DE); UPHUES, Günter, D-4019 Monheim (DE)
(86) Internationale Anmeldenummer: EP8901490
(87) Internationale Veröffentlichungsnummer: WO9006912

(56) Entgegenhaltungen:
- EP-A- 0 001 006
- DE-A- 2 725 780

## Beschreibung

Die Erfindung betrifft ein neues Verfahren zur Herstellung von amphoteren Tensidprodukten der Formel I 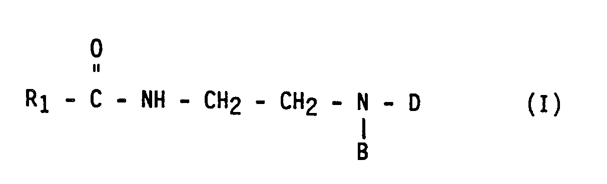
in der R₁ einen Alkylrest mit 5 bis 21 Kohlenstoffatomen, B einen Aminoalkyl- oder Hydroxyalkylrest mit 2 bis 4 Kohlenstoffatomen und D einen - R₂ - COOM - Rest bedeuten, wobei R₂ für eine Alkylenrest mit 1 bis 4 Kohlenstoffatomen oder einen Alkenylenrest mit 2 bis 4 Kohlenstoffatomen und M für Wasserstoff, Ammonium oder ein Alkalimetall steht. Diese Tenside ermöglichen im Gemisch mit anionischen Tensiden die Herstellung von Fertigformulierungen für die persönliche Pflege und Hygiene mit sehr geringer hautreizender Wirkung. Mit geringen Mengen herkömmlicher Verdikkungsmittel ermöglichen sie ohne weiteres die Einstellung der Viskosität, die für handelsübliche Fertigprodukte dieser Art erwünscht ist.

Das Verfahren besteht im wesentlichen aus der Hydrolyse eines Imidazolins der Formel II 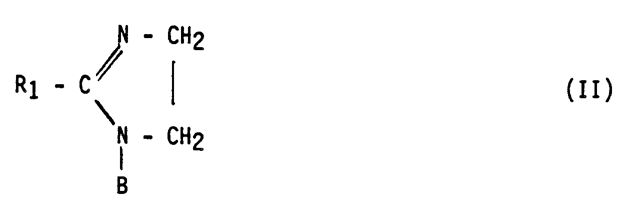
in der R₁ und B die oben angegebene Bedeutung haben, unter kontrollierten Bedingungen zu einem intermediären Amidoamin der Formel III, 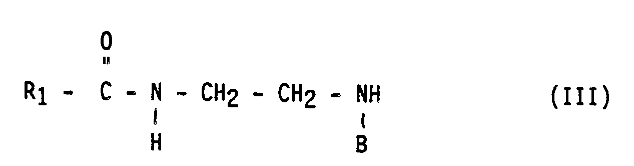
in der R₁ und B die oben angegebene Bedeutung haben, und der anschließenden Alkylierung dieses Amidoamins mit einem Alkylierungsmittel der Formel V,

X - D (V)

in der X für ein Halogenatom oder ein Wasserstoffatom steht und D die oben angegebene Bedeutung hat, unter Zugabe einer Base zur Verschiebung des Alkylierungsgleichgewichtes in die gewünschte Richtung durch Neutralisation der entstehenden Halogenwasserstoffssäure Bei dieser Base kann es sich um Ammoniumhydroxid oder um ein beliebiges Alkalihydroxid, vorzugsweise Natriumhydroxid, handeln. All das zielt in erster Linie auf die Bildung der Endverbindung der Formel I ab.

Bei den Imidazolinverbindungen der Formel II, die als Ausgangsmaterial für das erfindungsgemäße Verfahren in Betracht kommen, handelt es sich um bekannte Stoffe, die nach bekannten Verfahren der organischen Synthese, beispielsweise gemäß der EP-PS 2943 und der ES-PS 540 640 durch Reaktion einer Fettsäure der Formel R₁-COOH oder eines Fettsäuremethylesters der Formel R₁ - COOCH₃ mit einem Diamin der Formel H₂N - CH₂ - CH₂ - NH - B hergestellt werden können.

Diese Fettsäuren und die Fettsäurereste der in Frage kommenden Methylester können natürlicher oder synthetischer Herkunft sein. Als Ausgangsmaterial kommen beispielsweise Capron-, Heptan-, Capryl-, Undecyl-, Laurin-, Myristin-, Palmitin-, Stearin-, Behen-, Arachin-, Öl-, Linol-, Ricinol-, Ethylhexan-, und Isostearinsäure sowie deren Ester in betracht. Dabei können die einzelne Fettsäuren oder Ester einzelner Fettsäuren für die Synthese der Imidazoline verwendet werden. Es können aber auch Fettsäure- und Fettsäureestergemische eingesetzt werden, wie sie durch Fettspaltung oder Umesterung mit Methanol direkt aus natürlich vorkommenden Fetten und Ölen beispielsweise aus Kokosöl, erhältlich sind.

Als Alkylierungsmittel der Formel V kommen insbesondere Halogenalkansäuren (X = Halogen; R₂ = Alkylenrest mit 1 bis 4 Kohlenstoffatomen), vorzugsweise Monochloressigsäure, und Alkensäuren (X = Wasserstoff; R₂ = Alkenylrest mit 2 bis 4 Kohlenstoffatomen), vorzugsweise Acrylsäure, und deren Salze, insbesondere deren Alkalisalze, in Betracht. Natriummonochloracetat ist als Alkylierungsmittel besonders bevorzugt.

Bei den Diaminen der oben angegebenen Formel handelt es sich um solche, in denen B für einen Hydroxyalkyl- oder Aminoalkylrest steht, wobei B vorzugsweise einen Hydroxyalkylrest mit 2 bis 4 Kohlenstoffatomen, insbesondere einen Hydroxyethylrest bedeutet.

Bei den in der EP-PS 1006 und der ES-PS 540 508 beschriebenen Verfahren zur Herstellung von amphoteren Tensiden der hier zu betrachtenden Art wird ein Imidazolin der Formel II direkt mit einer wässrigen Lösung eines Alkylierungsmittels (V) mittels einer Base zur Reaktion gebracht. In diesen Verfahren wird gleichzeitig die Hydrolyse des Imidazolins und seine Alkylierung bewirkt. Es entsteht ein Gemisch aus Mono- und Dialkylamidoaminen und im allgemeinen wird ein Produkt mit niederer Viskosität erhalten. Wie bereits oben erwähnt, wird das Imidazolin schon zum Teil alkyliert, bevor durch seine Hydrolyse ein Gemisch aus Amidoaminen mit linearen Ketten entsteht, an denen die Alkylierung dann weitergeht.

Das Endprodukt besteht aus einer komplexen Mischung von alkylierten Verbindungen, die zum Teil durch Alkylierung des Imidazolins, anschließende hydrolytische Spaltung und weitere Alkylierung der linearen Hydrolyseprodukte, zum anderen Teil durch direkte Hydrolyse des Imidazolins und Alkylierung der linearen Amidoamine entstanden sind. Die Zusammensetzung des Endproduktes hängt von den Temperatur- und pH-Bedingungen ab, unter denen die Zugabe sowohl des Imidazolins als auch des Alkalihydroxids erfolgt. Arbeitet man bei einem hohen pH-Wert, so hat die Hydrolyse des Imidazolins den Vorrang vor der direkten Alkylierung und es entstehen Produkte der Formel I 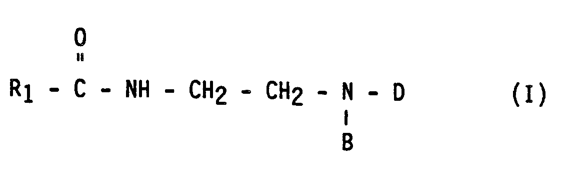
in der B und D die oben angegebene Bedeutung haben. Beim Arbeiten im technischen Maßstab ist es jedoch sehr schwierig, unter den vorgenannten Bedingungen auf Dauer Endprodukte zu erhalten, deren Viskositäten sich innerhalb eines akzeptablen engen Schwankungsbereichs bewegen.

Arbeitet man andererseits bei pH-Werten in der Nähe des Neutralpunktes oder im mäßig alkalischen Bereich, beispielsweise bei pH 7 bis 11, so ist die Geschwindigkeit der Alkylierung des Imidazolins größer als die seiner Hydrolyse. Es entsteht ein hochalkyliertes Endprodukt, das eine niedere Viskosität aufweist und im wesentlichen aus einer Mischung von Verbindungen der Formeln VI bis VIII besteht, 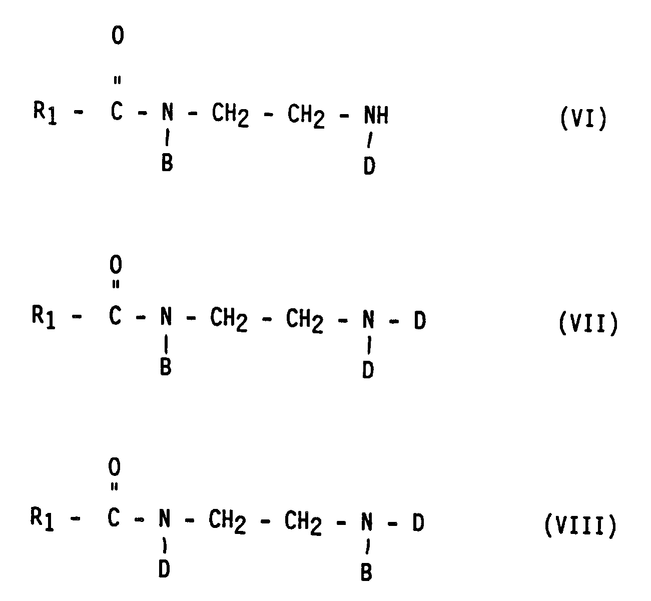
in denen R₁, B und D wieder die oben angegebene Bedeutung haben. Nach dem Ende der Reaktion muß ein gegebenenfalls vorhandener Überschuß an Alkylierungsmittel so hydrolysiert werden, daß bei der Verwendung des Endproduktes keine unerwünschten Effekte, beispielsweise starke Hautreizung, auftreten. Wenn beispielsweise Natriummonochloracetat als Alkylierungsmittel eingesetzt wird, entsteht bei dessen Hydrolyse Natriumchlorid und Natriumglycolat. Bei diesem Hydrolyseprozeß werden auch die tertiären Amide der Formeln (VI) bis (VIII) angegriffen, wobei unerwünschte Alkaliseifen entstehen, die im Endprodukt zu einer Erhöhung der Viskosität führen und, noch schwerwiegender, die Verträglichkeit des Produktes mit hartem Wasser stark beeinträchtigen.

Ein weiterer Nachteil der auf diesem Wege hergestellten Produkte besteht darin, daß sie große Mengen Verdickungsmittel, beispielsweise Polyethylenglycoldistearat 6000, erfordern, wenn sie zusammen mit Alkylsulfaten der Formel IX oder Alkylethersulfaten der Formel X

R₂ - O - SO₃ - M (IX)

R₂ - (OCH₂CH₂)n - O - SO₃M (X)

in denen R₂ einen Alkylrest mit 10 bis 18 Kohlenstoffatomen, n eine Zahl von 1 bis 10 und M ein Alkalimetall, vorzugsweise Natrium bedeutet, formuliert werden, damit in den Fertigprodukten die Viskositäten erzielt werden, die der Markt für diese Erzeugnisse verlangt.

Faßt man die Nachteile des bisherigen Standes der Technik zusammen, so läßt sich feststellen, daß nach den bekannten Verfahren entweder Produkte mit hoher aber schlecht reproduzierbarer Viskosität erhalten werden, oder Produkte mit niederer Viskosität, die einerseits bei der praktischen Formulierung erhebliche Mengen an Verdickungsmitteln erfordern, um in den Fertigprodukten ausreichende Viskositätswerte zu erzielen, und die andererseits durch ihren erheblichen Gehalt an Alkalimetallseifen zu einer Stabilitätseinbuße gegenüber hartem Wasser führen.

Gegenstand der Erfindung ist ein Verfahren zur Herstellung von Gemischen amphoterer grenzflächenaktiver Imidazolinderivate hoher bis mittlerer Viskosität, die hauptsächlich Verbindungen der Formel I enthalten, 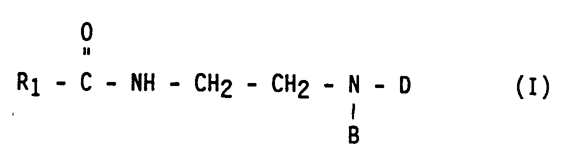
in der R₁ einen Alkylrest mit 5 bis 21 Kohlenstoffatomen, B einen Aminoalkyl- oder Hydroxyalkylrest mit 2 bis 4 Kohlenstoffatomen, vorzugsweise einen Hydroxyethylrest, und D einen -R₂-COOM-Rest bedeuten, wobei R₂ für einen Alkylenrest mit 1 bis 4 Kohlenstoffatomen oder einen Alkenylenrest mit 2 bis 4 Kohlenstoffatomen und M für Wasserstoff, Ammonium oder ein Alkalimetall, vorzugsweise Natrium, steht. Das neue Verfahren ist dadurch gekennzeichnet, daß man
A) ein Imidazolin der Formel II, 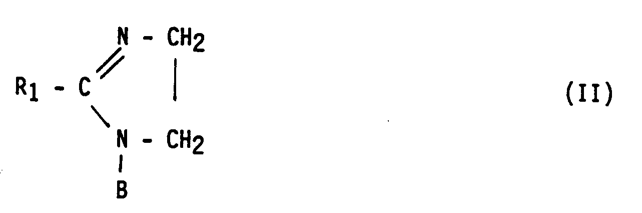 in der R₁ und B die oben angegebene Bedeutung haben, mit Wasser in einem Molverhältnis von 1 : 8 bis 1 : 20 bei Temperaturen zwischen 50 und 130°C unter atmosphärischem Druck oder geringem Überdruck im Verlauf von 1 bis 24 Stunden zu einem Gemisch von Amidoaminen der Formeln III und IV 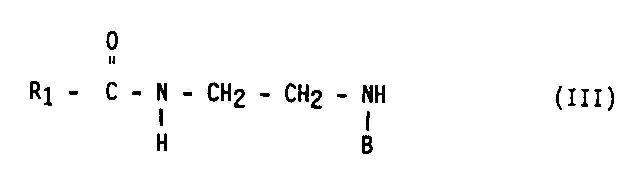 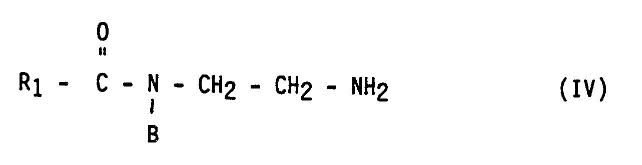 in denen R₁ und B die oben angegebene Bedeutung haben, hydrolysiert,
B) das Gemisch der erhaltenen Amidoamine mit einem Alkylierungsmittel der Formel V,

   X - D (V)

   in der X für ein Halogenatom, vorzugsweise ein Chloratom, oder ein Wasserstoffatom steht und D die oben angegebene Bedeutung hat, in einem Molverhältnis von Alkylierungsmittel (V) zu Amidoaminen (III + IV) zwischen 1,5 : 1 und 2,5 : 1 mit Hilfe einer Base bei 40 bis 70°C unter Einhaltung eines pH-Wertes zwischen 10 und 11,5, gemessen in einer 10 gew.-%igen wässrigen Lösung des Reaktionsgemisches, alkyliert, so daß in erster Linie die Endverbindung der Formel I und in geringerem Maße auch Verbindungen der Formeln VI und VII entstehen, 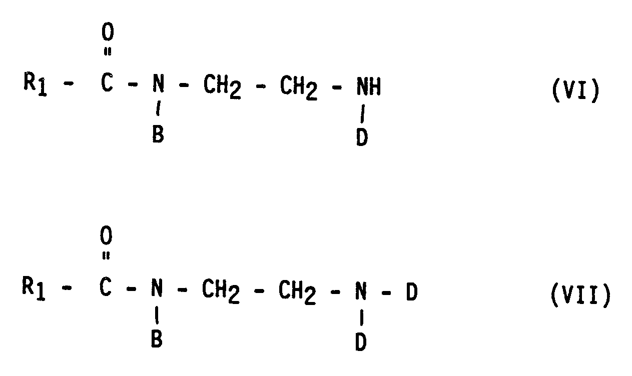 in denen R₁, und D die oben angegebene Bedeutung haben, und anschließend
C) das überschüssige Alkylierungsmittel der Formel V bei einer Temperatur zwischen 80 und 90°C unter Einhaltung eines pH-Wertes zwischen 10 und 11,5, gemessen in einer 10 gew.-%igen wässrigen Lösung des Reaktionsgemisches, hydrolysiert.

Die mit Hilfe des erfindungsgemäßen Verfahrens hergestellten Produkte weisen von sich aus eine hohe Viskosität auf, wodurch die anwendungstechnischen Nachteile, mit denen die Produkte des Standes der Technik behaftet sind, in erheblichem Maß reduziert werden. Mit Hilfe des neuen Verfahrens können Produkte mit hohen, jedoch nicht übermäßigen Viskositäten im Bereich von 1000 bis 5000 cP hergestellt werden. Diese Produkte können ohne weiteres in den industrieüblichen Anlagen gehandhabt und gefördert werden. Weiterhin kann die Menge des Verdickungsmittels, das erforderlich ist, um die vom Markt geforderten Viskositätswerte bei Fertigprodukten, beispielsweise Shampoos, einzustellen, erheblich reduziert werden. Schließlich ist der Gehalt an störenden Metallseifen in den nach dem erfindungsgemäßen Verfahren erhaltenen Produkten wesentlich geringer als in den nach den bekannten Verfahren hergestellten Produkten.

Überraschender Weise wird durch die gezielte Hydrolyse des Imidazolins vor der Alkylierung erreicht, daß die Zusammensetzung des Endprodukts kontrolliert und so ausgerichtet werden kann, daß die oben erwähnten Eigenschaften erzielt werden.

GB-A- 850 514 offenbart ein Verfahren in dem ein Imidazolin gemäß dem vorliegenden Anspruch 1 zunächst hydrolisiert, und erst anschließend alkyliert wird. In diesem Verfahren wird jedoch ein anderes Verhältnis von Imidazolin zu Wasser bei der Hydrolyse eingesetzt.

Die Herstellung des intermediären Imidazolins der Formel II 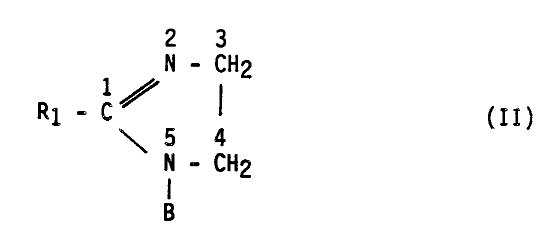
in der R₁ und B₁ die oben angegebene Bedeutung haben, ist hinreichend bekannt und beispielsweise in der ES-PS 540 640 und der EP-PS 2943 beschrieben.

Die Hydrolyse des Imidazolins der Formel II kann zwei verschiedene Amidoamintypen hervorbringen. Das Amidoamin der Formel III 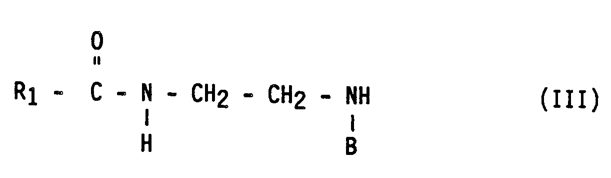
entsteht, wenn die Hydrolysespaltung zwischen dem Kohlenstoffatom 1 und dem Stickstoffatom 5 des Imidazolinringes eintritt. Das Amidoamin der Formel IV 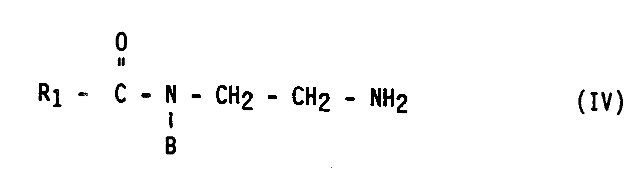
entsteht, wenn die Hydrolysespaltung zwischen dem Kohlenstoffatom 1 und dem Stickstoffatom 2 des Imidazolinringes eintritt.

Es wurde überraschenderweise festgestellt, daß bei der Hydrolyse des Imidazolins eine totale Spaltung der -C = N-Bindung abläuft und zwar nach einem kinetischen Mechanismus, in dem die Bildung des Amidoamins der Formel IV bevorzugt ist. Daneben läuft ein langsamer Isomerisierungsprozeß ab, bei dem das Amidoamin der Formel IV unter Bildung des Amidoamins der Formel III verschwindet, welch letzteres thermodynamisch stabiler ist. Es ist deshalb bei der Hydrolyse möglich, durch Steuerung der Temperatur- und Zeitbedingungen das gewünschte Verhältnis zwischen den beiden Amidoamintypen (III) und (IV) im Hydrolyseprodukt einzustellen. Die Eigenschaften des Endproduktes sind optimal, wenn in der Hydrolyse des Imidazolins ein Anteil an Amidoaminen der Formel III von 50 bis 95 Gew.-%, vorzugsweise von 80 bis 90 Gew.-%, bezogen auf das gesamte Hydrolyseprodukt, erreicht wird.

Wenn es möglich wird, die Zusammensetzung des intermediären Hydrolyseproduktes zu steuern und festzulegen, dann kann man damit auch ein vollkommen definiertes Endprodukt erhalten. Im konkreten Fall werden bei der Alkylierung mit einem Alkylierungsmittel der Formel X-D aus dem Amidoamin der Formel IV die Verbindungen der Formeln VI und VII erhalten. Die Alkylierung des Amidoamins der Formel III hingegen führt zu dem Alkylierungsprodukt der Formel I, das im allgemeinen den größten Teil des Endproduktes ausmacht.

Die Hydrolyse des Imidazolins der Formel II kann bei atmosphärischem Druck oder leichtem Überdruck in einem Temperaturbereich zwischen 50 und 130 °C, vorzugsweise zwischen 80 und 90 °C und mit einem Molverhältnis von Imidazolin zu Wasser zwischen 1 : 8 bis 1 : 20, vorzugsweise zwischen 1 : 8 bis 1 : 15 durchgeführt werden. Dabei ist zu berücksichtigen, daß die Bildung des sekundären Amidoamins der Formel III zu Lasten des tertiären Amidoamins der Formel IV durch lange Hydrolysezeiten, insbesondere Hydrolysezeiten zwischen 1 und 24 Stunden, vorzugsweise zwischen 6 und 15 Stunden, und hohe Temperaturen, insbesondere Temperaturen zwischen 50 und 130 °C, vorzugsweise zwischen 90 und 110 °C, begünstigt wird.

Die Alkylierung der Hydrolyseprodukte erfolgt wie oben bereits angegeben nach bekannten Verfahren. Dabei wird das in der vorangegangenen Hydrolysestufe erhaltene Gemisch der Amidoamine einer wäßrigen Lösung des Alkylierungsmittels, vorzugsweise Natriummonochloracetat, bei einer Temperatur zwischen 40 und 70 °C, vorzugsweise zwischen 45 und 55 °C, zugesetzt. Das Molverhältnis von Amidoaminen zu Alkylierungsmitteln liegt dabei zwischen 1 : 1 und 4 : 1, vorzugsweise zwischen 1,5 : 1 und 2,8 : 1. Anschließend wird allmählich eine wässrige Alkalihydroxidlösung, vorzugsweise Natriumhydroxidlösung, unter Beibehaltung der angegebenen Temperaturwerte mit einer solchen Geschwindigkeit zugegeben, daß der pH-Wert, gemessen in einer 10 gew.-%igen Lösung des Reaktionsgemisches, zwischen 10 und 11,5, vorzugsweise zwischen 10,8 und 11,2, gehalten wird und die Alkylierung den vorhandenen Aminstickstoffatomen in einem angemessenen Zeitraum von etwa 3 bis 6 Stunden stattfindet. Die genannten Bedingungen schaffen gleichzeitig die Möglichkeit, in dieser Phase des Prozesses die Geschwindigkeit der Hydrolyse des Alkylierungsmittels entsprechend der Gleichung

ClCH₂COOONa + NaOH → NaCl + HOCH₂COONa

auf einen niedrigen Wert einzustellen. Wenn der gewünschte Alkylierungsgrad erreicht ist, wird die Temperatur des Reaktionsgemisches auf 80 bis 95 °C, vorzugsweise auf 85 bis 90 °C, erhöht. Das Reaktionsgemisch wird dann unter laufender Zugabe von Natriumhydroxidlösung zur Aufrechterhaltung eines pH-Wertes innerhalb der oben angegebenen Grenzen so lange auf dieser Temperatur gehalten, bis das überschüssige Alkylierungsmittel zerstört ist.

### Beispiele

### Beispiel 1

In einem mit Heizung und mechanischer Rührvorrichtung versehenen Labor-Glasreaktionsgefäß wurden 165,6 g (0,62 Mol) Hydroxyethylalkylimidazolin (R₁ = C₁₁; über 95 Gew.-% Imidazolingehalt) vorgelegt und unter Rühren auf 80 °C erwärmt. In einem Zeitraum von einer halben Stunde wurden 128 g (7,1 Mol) destilliertes Wasser zudosiert. Die Mischung wurde unter Rühren 15 Stunden lang zum Rückflußkochen erhitzt, so daß durch Hydrolyse eine wässrige Lösung eines Gemisches von Amidoaminen erhalten wurde, in dem bezogen auf Trockensubstanz, 83 Gew.-% sekundäres Amidoamin (III) enthalten waren.

In einem zweiten Labor-Glasreaktionsgefäß, das ebenso ausgestattet war wie das vorgenannte, wurden 143,6 g (1,23 Mol) Natriummonochloracetat in 482,5 g destilliertem Wasser gelöst. Diese Lösung wurde auf 50 °C erwärmt. Danach wurde unter Rühren innerhalb einer halben Stunde die oben beschriebene Mischung aus Amidoaminen zudosiert. Anschließend wurde 3 Stunden lang unter Beibehaltung der Temperatur von 50 °C und unter weiterem Rühren 64 g einer 50 gew.-%igen wässrigen Natriumhydroxydlösung so zudosiert, daß der pH-Wert einer 10 gew.-%igen wässrigen Lösung des Reaktionsgemisches zwischen 11,1 und 11,5 lag. Schließlich wurde die Temperatur auf 90 °C erhöht und im Verlauf von 5 Stunden 16 g 50 gew.-%ige Natriumhydroxidlösung zudosiert, so daß der pH-Wert einer 10 gew.-%igen wässrigen Lösung des Reaktionsgemisches zwischen 10,0 und 10,5 lag. Das Endprodukt hatte einen Wassergehalt von 65 Gew.-% und bei 20 °C eine Viskosität von 1500 cP, gemessen mit einem Brookfield-Viskosimeter.

### Beispiel 2

In das im Beispiel 1 beschriebene Labor-Glasreaktionsgefäß wurden 133 g (0,5 Mol) Hydroxyethylalkylimidazolin (R₁ = C₁₁; über 95 Gew.-% Imidazolingehalt) vorgelegt und auf 105 °C erwärmt. Unter Rühren wurden innerhalb einer halben Stunde 103 g (5,7 Mol) destilliertes Wasser zudosiert. Die Mischung wurde unter Rühren 2 Stunden lang zum Rückflußkochen erhitzt, so daß eine wässrige Lösung eines Gemisches von Amidoaminen erhalten wurde, in dem, bezogen auf Trockensubstanz, 73 Gew.-% Amidoamin (III) enthalten waren.

Die Alkylierung erfolgte unter den gleichen Bedingungen, wie sie im Beispiel 1 beschrieben sind, dabei wurden 155,8 g (1,3 Mol) Natriummonochloracetat, 107 g 50 gew.-%ige wässrige Natriumhydroxidlösung und 502 g destilliertes Wasser eingesetzt.

Das Endprodukt hatte einen Wassergehalt von 65 Gew.-% und bei 20 °C eine Viskosität von 180 cP, bestimmt mit einem Brookfield-Viskosimeter.

### Beispiel 3

Mit den gleichen Reagentien und Mengen wie in Beispiel 2, jedoch unter Durchführung der Hydrolyse bei 105 °C im Verlauf von 6 Stunden, wurde eine wäßrige Lösung von Amidoaminen mit einem Gehalt an 96 Gew.-% sekundärem Amidoamin (III), bezogen auf Trokkensubstanz, erhalten.

Unter Verwendung der gleichen Reagentien in den gleichen Mengen und unter Einhaltung der gleichen Bedingungen wie im Beispiel 2 wurde ein Endprodukt mit einem Wassergehalt von 65 Gew.-% und einer Viskosität bei 20 °C von 5800 cP, gemessen mit einem Brookfield-Viskosimeter, erhalten.

### Beispiel 4

Mit den gleichen Reagentien in gleichen Mengen und unter den gleichen Bedingungen wie im Beispiel 1, jedoch unter Verwendung eines Alkylhydroxyethylimidiazolins der Formel I, dessen Substituenten R₁ aus einem Gemisch von Alkylresten mit 7 bis 17 Kohlenstoffatomen, abgeleitet aus dem Fettsäureanteil des Kokosöl, wurde ein Endprodukt mit einem Wassergehalt von 65 Gew.-% und einer Viskosität bei 20 °C von 1200 cP, bestimmt mit einem Brookfield-Viskosimeter, erhalten.

## Patentansprüche

1. Verfahren zur Herstellung von Gemischen amphoterer grenzflächenaktiver Imidazolinderivate hoher bis mittlerer Viskosität, die hauptsächlich Verbindungen der Formel I enthalten, 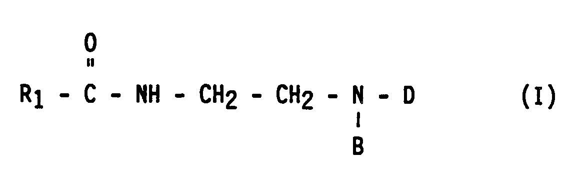 in der R₁ einen Alkylrest mit 5 bis 21 Kohlenstoffatomen, B einen Aminoalkyl- oder Hydroxyalkylrest mit 2 bis 4 Kohlenstoffatomen, vorzugsweise einen Hydroxyethylrest, und D einen -R₂-COOM-Rest bedeuten, wobei R₂ für einen Alkylenrest mit 1 bis 4 Kohlenstoffatomen oder einen Alkenylenrest mit 2 bis 4 Kohlenstoffatomen und M für Wasserstoff, Ammonium oder ein Alkalimetall, vorzugsweise Natrium, steht, dadurch gekennzeichnet, daß man
A) ein Imidazolin der Formel II, 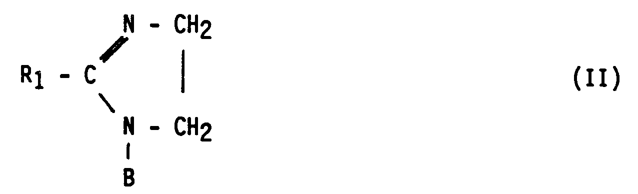 in der R₁ und B die oben angegebene Bedeutung haben, mit Wasser in einem Molverhältnis von 1 : 8 bis 1 : 20 bei Temperaturen zwischen 50 und 130°C unter atmosphärischem Druck oder geringem Überdruck im Verlauf von 1 bis 24 Stunden zu einem Gemisch von Amidoaminen der Formeln III und IV 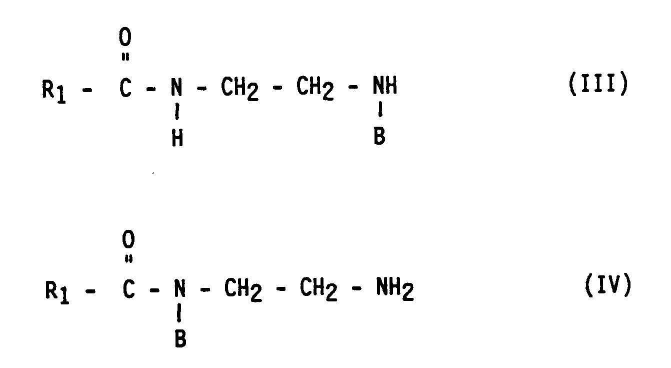 in denen R₁ und B die oben angegebene Bedeutung haben, hydrolysiert,
B) das Gemisch der erhaltenen Amidoamine mit einem Alkylierungsmittel der Formel V,
X - D (V)
in der X für ein Halogenatom, vorzugsweise ein Chloratom oder ein Wasserstoffatom, steht und D die oben angegebene Bedeutung hat, in einem Molverhältnis von Alkylierungsmittel (V) zu Amidoaminen (III + IV) zwischen 1 : 1 und 4 : 1 mit Hilfe einer Base bei 40 bis 70°C unter Einhaltung eines pH-Wertes zwischen 10 und 11,5, gemessen in einer 10 gew.-%igen wässrigen Lösung des Reaktionsgemisches, alkyliert, so daß in erster Linie die Endverbindung der Formel I und in geringerem Maße auch Verbindungen der Formeln VI und VII entstehen, 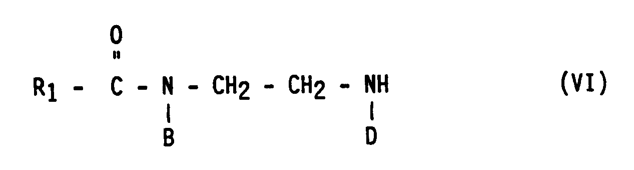 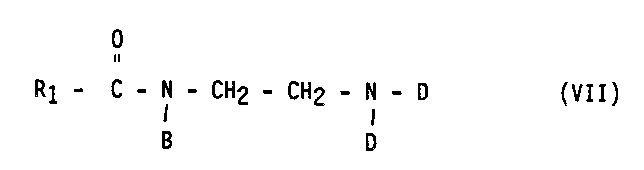 in denen R₁, und D die oben angegebene Bedeutung haben, und anschließend
C) das überschüssige Alkylierungsmittel der Formel V bei einer Temperatur zwischen 80 und 95°C unter Einhaltung eines pH-Wertes zwischen 10 und 11,5, gemessen in einer 10 gew.-%igen wässrigen Lösung des Reaktionsgemisches, hydrolysiert.

2. Verfahren nach Anspruch 1, dadurch gekennzeichnet, daß in den Formeln I, V, VI und VII R₂ in D einen Ethylenrest und vorzugsweise einen Methylenrest bedeutet.

3. Verfahren nach den Ansprüchen 1 und 2, dadurch gekennzeichnet, daß bei der Hydrolyse das Molverhältnis von Imidazolin (II) zu Wasser zwischen 1 : 8 bis 1 : 15 liegt.

4. Verfahren nach den Ansprüchen 1 bis 3, dadurch gekennzeichnet, daß die Hydrolyse des Imidazolins (II) bei einer Temperatur zwischen 90 und 110°C durchgeführt wird.

5. Verfahren nach den Ansprüchen 1 bis 4, dadurch gekennzeichnet, daß die Hydrolyse des Imidazolins (II) im Verlauf von 6 bis 15 Stunden durchgeführt wird.

6. Verfahren nach den Ansprüchen 1 bis 5, dadurch gekennzeichnet, daß die Alkylierung bei einer Temperatur zwischen 45 und 55°C und bei einem pH-Wert zwischen 10,8 und 11,2, gemessen in einer 10 gew.-%igen Lösung des Reaktionsgemisches, durchgeführt wird.

7. Verfahren nach den Ansprüchen 1 bis 6, dadurch gekennzeichnet, daß bei der Alkylierung das Molverhältnis von Amidoaminen zu Alkylierungsmittel zwischen 21,5 : 1 und 2,8 : 1 liegt.

8. Verfahren nach den Ansprüchen 1 bis 7, dadurch gekennzeichnet, daß die Hydrolyse des überschüssigen Alkylierungsmittels (V) bei 85 bis 90°C unter Einhaltung eines pH-Wertes zwischen 10,8 und 11,2, gemessen in einer 10 gew.-%igen wässrigen Lösung des Reaktionsgemisches, durchgeführt wird.

## Claims

1. A process for the production of mixtures of amphoteric interfacially active imidazoline derivatives of high to medium viscosity which mainly contain compounds corresponding to formula I: 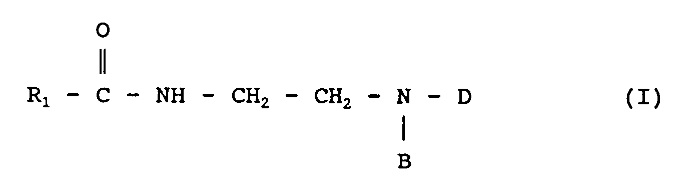 in which R₁ is an alkyl radical containing 5 to 21 carbon atoms, B is an aminoalkyl or hydroxyalkyl radical containing 2 to 4 carbon atoms, preferably a hydroxyethyl radical, and D is an -R₂-COOM group, where R₂ is an alkylene radical containing 1 to 4 carbon atoms or an alkenylene radical containing 2 to 4 carbon atoms and M is hydrogen, ammonium or an alkali metal, preferably sodium, characterized in that
A) an imidazoline corresponding to formula II: 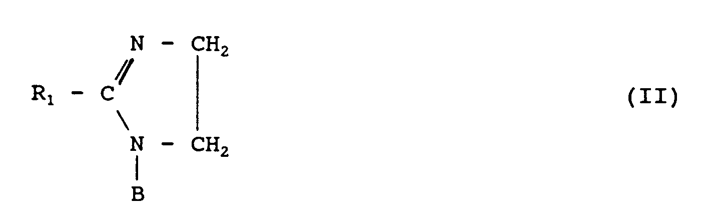 in which R₁ and B are as defined above, is hydrolyzed with water in a molar ratio of 1:8 to 1:20 over a period of 1 to 24 hours at temperatures of 50 to 130°C and under atmospheric pressure or a slight excess pressure to form a mixture of amidoamines corresponding to formulae III and IV: 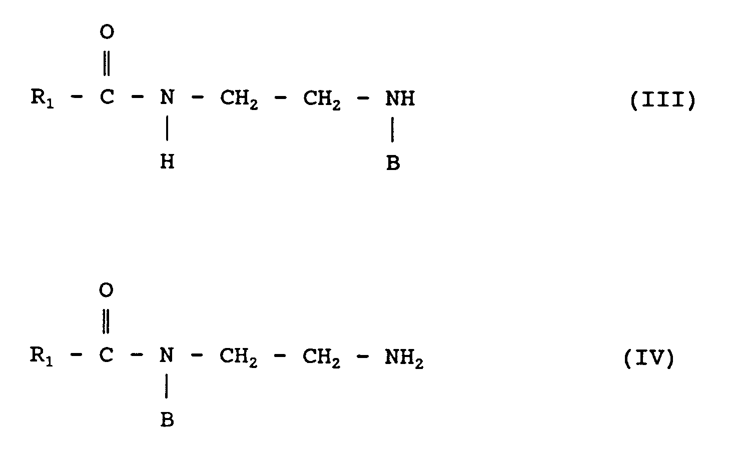 in which R₁ and B are as defined above,
B) the mixture of the amidoamines obtained is alkylated with an alkylating agent corresponding to formula V:
X - D (V)
in which X is a halogen atom, preferably a chlorine atom, or a hydrogen atom and D is as defined above, in a molar ratio of alkylating agent (V) to amidoamines (III + IV) or 1:1 to 4:1 in the presence of a base at 40 to 70°C and at a pH value of 10 to 11.5, as measured on a 10% by weight agueous solution of the reaction mixture, so that primarily the final compound corresponding to formula I and, to a lesser extent, compounds corresponding to formulae VI and VII are formed: 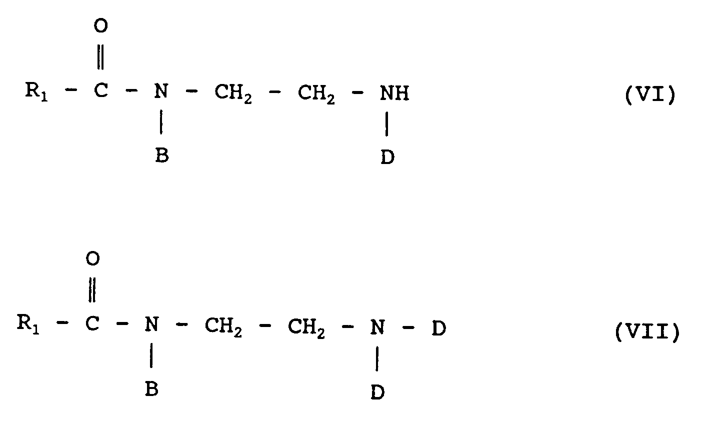 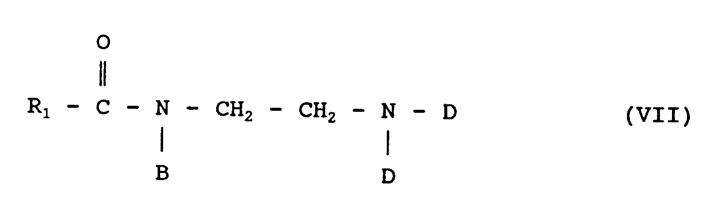 in which R₁ and D are as defined above, after which
C) the excess alkylating agent corresponding to formula V is hydrolyzed at a temperature of 80 to 95°C and at a pH value of 10 to 11.5, as measured on a 10% by weight aqueous solution of the reaction mixture.

2. A process as claimed in claim 1, characterized in that, in formulae I, V, VI and VII, R₂ in D is an ethylene radical and preferably a methylene radical.

3. A process as claimed in claims 1 and 2, characterized in that the molar ratio of imidazoline (II) to water in the hydrolysis step is 1:8 to 1:15.

4. A process as claimed in claims 1 to 3, characterized in that the hydrolysis of the imidazoline (II) is carried out at a temperature of 90 to 110°C.

5. A process as claimed in claims 1 to 4, characterized in that the hydrolysis of the imidazoline (II) is carried out over a period of 6 to 15 hours.

6. A process as claimed in claims 1 to 5, characterized in that the alkylation is carried out at a temperature of 45 to 55°C and at a pH value of 10.8 to 11.2, as measured on a 10% by weight solution of the reaction mixture.

7. A process as claimed in claims 1 to 6, characterized in that the molar ratio of amidoamines to alkylating agent in the alkylation step is between 21.5:1 and 2.8:1.

8. A process as claimed in claims 1 to 7, characterized in that the hydrolysis of the excess alkylating agent (V) is carried out at 85 to 90°C at a pH value of 10.8 to 11.2, as measured on a 10% by weight aqueous solution of the reaction mixture.

## Revendications

1. Procédé de production de mélanges de dérivés dimidazoline amphotères tensioactifs de viscosité haute à moyenne, qui contiennent principalement des composés de la formule I 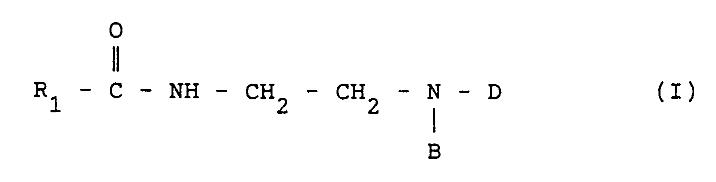 dans laquelle R₁ est un alkyle avec de 5 à 21 atomes de carbone, B représente un radical aminoalkyle ou hydroxyalkyle avec de 2 à 4 atomes de carbone, de préférence un radical hydroxyéthyle, et D est un radical - R₂ - COOM - dans lequel R₂ étant un alkyle avec de 1 à 4 atomes de carbone ou un alcényle avec de 2 à 4 atomes de carbone et M est un hydrogène, un ammonium ou un métal alcalin, de préférence du sodium, caractérisé en ce que :
A) on hydrolyse une imidazoline de formule II. 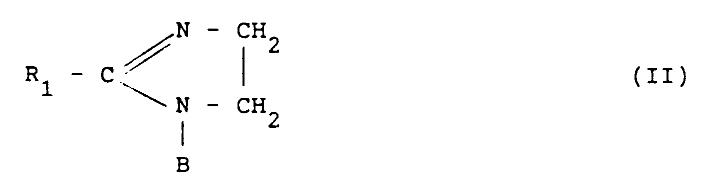 dans laquelle R₁ et B ont la signification données plus haut, avec de l'eau dans un rapport molaire de 1 : 8 à 1 ; 20 à des températures entre 50 et 130°C sous pression atmosphériques ou sous une faible pression effective au cours de 1 à 24 heures, en un mélange d'amidoamines des formules III et IV. 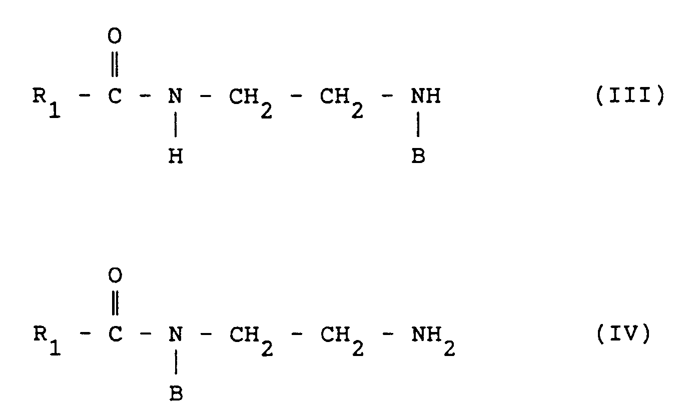 dans lesquelles R₁ et B ont la signification indiquée plus haut,
B) on alkyle le mélange d'amidoamine obtenu avec un agent d'alkylation de formule V,
X - D (V)
dans laquelle X signifie un atome d'halogène, de préférence un atome de chlore, ou un atome d'hydrogène et D a la signification indiquée plus haut,
dans un rapport molaire entre agent d'alkylation (V) et amidoamines (III + IV) compris entre 1 : 1 et 4 : 1 à l'aide d'une base entre 40 jusqu'à 70°C en maintenant une valeur de pH entre 10 et 11,5, mesurée dans une solution aqueuse à 10 % en poids du mélange réactionnel, de façon que prennent naissance en premier lieu le composé final de formule I et dans une faible mesure aussi des composés des formules VI et VII se forment, 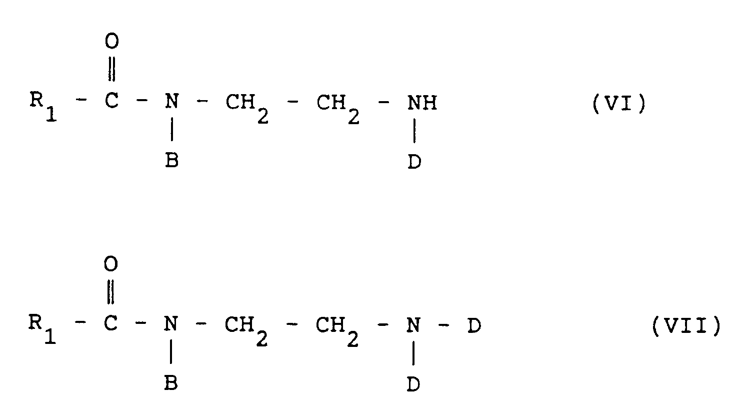 dans lesquelles R₁ et D ont la signification indiquée plus haut, et ensuite.
C) on hydrolyse l'agent d'alkylation restant de formule (V) à une température entre 80 et 95°C en maintenant une valeur de pH comprise entre 10 et 11,5, mesurée dans une solution aqueuse à 10 % en poids du mélange réactionnel.

2. Procédé selon la revendication 1, caractérisé en ce que dans les formules I, V, VI et VII, R₂ dans D représente un radical éthyle et de préférence un radical méthyle.

3. Procédé selon les revendications 1 et 2, caractérisé en ce que lors de l'hydrolyse, le rapport molaire de l'imidazoline (II) à l'eau se situe entre 1 : 8 et 1 : 15.

4. Procédé selon les revendications 1 à 3, caractérisé en ce que l'hydrolyse de l'imidazoline (II) est effectuée à une température entre 90 et 110°C.

5. Procédé selon les revendications 1 à 4, caractérisé en ce que l'hydrolyse de l'imidazoline (II) est réalisée au cours de 6 à 15 heures.

6. Procédé selon les revendications 1 à 5, caractérisé en ce que l'alkylation est réalisée à une température comprise entre 45 et 55°C et à une valeur de pH entre 10,8 et 11,2, mesurée dans une solution à 10 % en poids du mélange réactionnel.

7. Procédé selon les revendications 1 à 6, caractérisé en ce que lors de l'alkylation, le rapport molaire des amidoamines à l'agent d'alkylation se situe entre 21,5 : 1 et 2,8 : 1.

8. Procédé selon les revendications 1 à 7, caractérisé en ce que l'hydrolyse de l'agent d'alkylation résiduel (V) est réalisé à 85 - 90° en maintenant une valeur de pH entre 10,8 et 11,2, mesurée dans une solution aqueuse à 10 % en poids du mélange réactionnel.
